# EUROPEAN PATENT SPECIFICATION

(11) **EP 4 098 305 B1**
(45) Date of publication and mention of the grant of the patent: **15.04.2026**
(21) Application number: 22176191.9
(22) Date of filing: 30.05.2022
(51) Int. Cl.: A61M 16/06, A61M 16/08, A61B 17/34, A61J 15/00

(54) **ACCESS DEVICE FOR A CATHETER AND/OR A NASOGASTRIC TUBE FOR A RESPIRATORY INTERFACE AND RELATED RESPIRATORY INTERFACE**
ZUGANGSVORRICHTUNG FÜR EINEN KATHETER UND/ODER EINE NASOGASTRALE SONDE FÜR EINE ATMUNGSSCHNITTSTELLE UND ENTSPRECHENDE ATMUNGSSCHNITTSTELLE
DISPOSITIF D'ACCÈS POUR UN CATHÉTER ET/OU UN TUBE NASOGASTRIQUE POUR UNE INTERFACE RESPIRATOIRE ET INTERFACE RESPIRATOIRE

(30) Priority: 01.06.2021 IT 202100014360
(43) Date of publication of application: 07.12.2022
(73) Proprietor: HMC Premedical S.p.A., 41037 Mirandola (MO) (IT)
(72) Inventor: Borsari, Maurizio, 41037 Mirandola (MO) (IT)
(74) Representative: Branca, Emanuela

(56) References cited:
- EP-A1- 2 548 600
- EP-A1- 2 684 578
- IT-A1- UB20 155 514
- US-A1- 2009 082 632

## Description

The present invention relates to an access device for a catheter and/or a nasogastric tube for a respiratory interface for non-invasive ventilation, as well as the related respiratory interface.

Medical devices for non-invasive ventilation, such as helmets or respiratory masks, frequently require the insertion of a nasogastric tube or other accessories, which are even large and bulky.

It is common practice to use tubes during non-invasive ventilation therapies.

The nasogastric tube may be used to:
- aspirate the gastric contents, even in case of intestinal obstruction;
- administer enteral nutrition;
- empty the stomach out of dangerous contents (gastric emptying or gastric lavage);
- prevent the distension of the stomach prior to or after surgery, although there is no recognition in literature regarding the effectiveness of this use.

There are different types of nasogastric tubes on the market, which differ in diameter and length.

The tube used for enteral feeding may be made of silicone or polyurethane because it must be soft, flexible so that its residence is little traumatic. In adult patients, tubes with a diameter between 8 and 12 French are used (1 French is equivalent to 0.3 mm), while in children, tubes with a diameter between 6 and 8 French are used.

Adult tubes can vary in diameter and length (90-145 cm).

For enteral feeding, magnetic-tipped tubes that easily pass through the pylorus may be useful. The positioning of these tubes can be checked easily and safely with no need of radiography. In Italy, however, these types of tubes are little used.

The tube used for drug administration and gastric decompression is larger (at least 14 French) and usually less flexible. There appears to be no significant difference in the risk of aspiration pneumonia with tubes of a different size.

There are also radiopaque and double-lumen tubes, such as the Salem tube used for continuous aspiration. The second lumen is for maintaining a constant pressure inside the stomach. To prevent gastric material from entering the lumen, the valves are unidirectional.

Another double-lumen device, with a calibre between 12 and 18 French, is the Miller-Abbott tube, which has a balloon at the distal end that inflates through a dedicated lumen and is used to advance the tube into the first digestive loops.

As the manoeuvre for introducing the tube is complex and unpleasant for the patient because of the discomfort it causes, the tendency is to leave the tube in place for the time required. It is not therefore feasible to remove and reintroduce it whenever a respiratory interface, such as a helmet or mask, needs to be fitted and/or replaced.

As the tube has to be kept in place in the patient, the main issue is the passage of the tube connection fitting, opposite the tip, through the medical ventilation device used by the patient.

Nowadays, fittings are used that are placed on the respiratory interface, helmet or mask, having elastic, expandable holes that allow for the passage of the tube fitting. Examples of this fittings are shown in IT UB20 155 514 A1, EP 2 684 578 A1, EP 2 548 600 A1.

This step is possible if a single-lumen tube is used, due to the small size thereof varying from 12 to 18 mm.

On the other hand, it is impossible to pass the fitting of other tubes such as double-lumen tubes or others, as they are considerably larger (28mm or larger) .

In case double-lumen tubes are used, to date, passing the body of the tube under the helmet sealing membrane placed between the patient's neck and chest, or between the patient's skin and the mask sealing membrane, inevitably results in leaks into the device, worsening both the patient's comfort and the pneumatic sealing of the system, with considerable problems of "synchronism" and "autotrigger" in the use in PSV and/or CPAP pressure support techniques.

The use of double-lumen nasogastric tubes, or those with a large connection fitting, is currently difficult in patients undergoing Non-Invasive Ventilation/CPAP due to the patient interfaces currently available on the market (helmets/masks), as the latter do not have access ports large enough to allow for the passage of the connection fitting and the subsequent pneumatic sealing of such tubes.

The object of the present invention is to make an access device for a catheter and/or a nasogastric tube for a medical device for non-invasive ventilation which solves the problem of the passage of the connection fitting of the tube, opposite the tip, through the medical device for non-invasive ventilation used by the patient, for tubes even of a significant diameter.

A further object of the present invention is to make an access device for a catheter and/or a nasogastric tube for a medical device for non-invasive ventilation that avoids "synchronism" and "autotrigger" problems when used in PSV and/or CPAP pressure support techniques.

A further object of the present invention is to make an access device for a catheter and/or a nasogastric tube for a medical device for non-invasive ventilation, which allows for the mechanical and pneumatic sealing of the system while improving the patient comfort.

A further object of the present invention is to make an access device for a catheter and/or a nasogastric tube for a medical device for non-invasive ventilation, that is particularly simple and functional and at a low cost.

These objects according to the present invention are reached by making an access device for a catheter and/or a nasogastric tube for a medical device for non-invasive ventilation as set forth in claim 1.

Further features are provided in the dependent claims.

The features and advantages of an access device for a catheter and/or a nasogastric tube for a medical device for non-invasive ventilation according to the present invention will be more apparent from the following description, which is illustrative and not limiting, referred to the enclosed schematic drawings in which:
figures 1 and 2 show a respiratory interface, consisting of a helmet, provided with an access device for a catheter and/or a nasogastric tube according to the present invention in an open and closed position respectively;
figures 3 and 4 show a respiratory interface, consisting of a mask, provided with an access device for a catheter and/or a nasogastric tube according to the present invention in an open and closed position respectively;
figure 5 is an exploded view of a first embodiment of an access device for a catheter and/or a nasogastric tube according to the present invention;
figure 6 shows in section some details of the device of figure 5 in a closed position, excluding the tubular;
figures 7 and 8 are respectively perspective views of a second embodiment of the access device of a catheter and/or a nasogastric tube according to the present invention in an open and closed position;

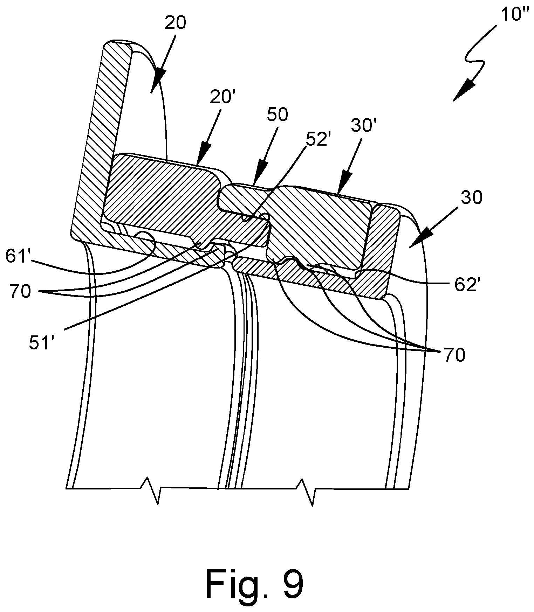

figure 9 shows in section some details of the device of figure 7 in a closed position, excluding the tubular;
figures 10 and 11 show in section the constraint of the tubular to the fixed flanges and movable flanges respectively.

Referring to the figures, it is shown an access device for a catheter and/or a nasogastric tube overall referred to as 10, 10' for a respiratory interface for non-invasive ventilation 100, 100'.

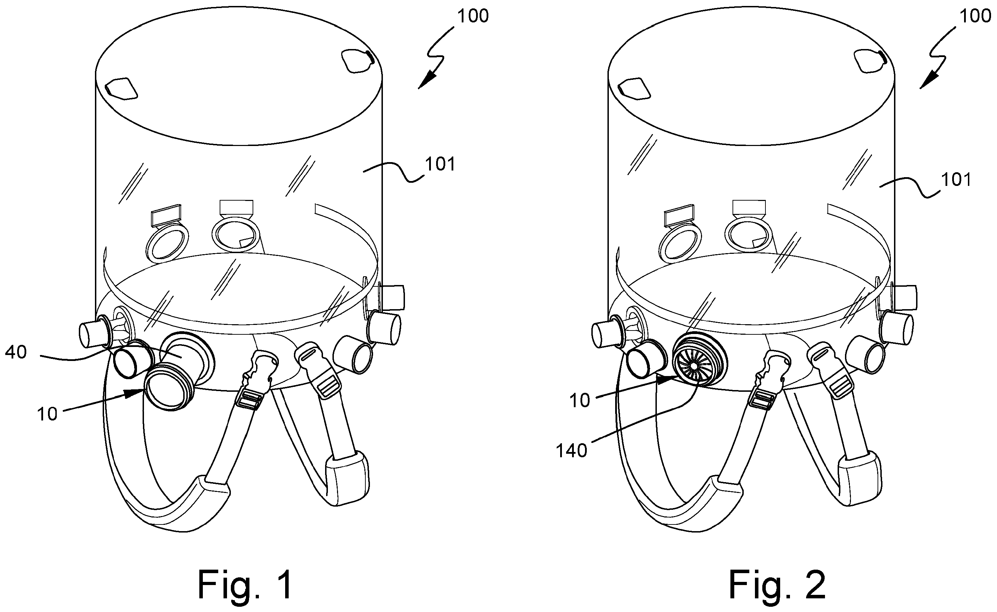

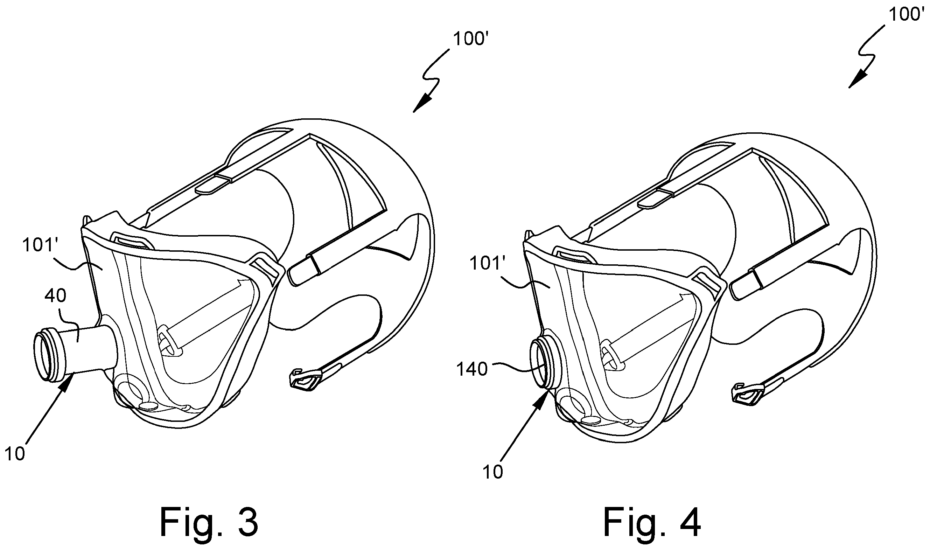

The illustrative and non-limiting images show the access device 10 applied to a helmet 100 (figures 1 and 2) or to a mask 100' (figures 3 and 4), according to two at all equivalent alternatives.

The point of application of the access device 10 on the respiratory interface may be any on the respiratory chamber 101, 101', on either flexible components or rigid components.

The access device for a catheter and/or a nasogastric tube 10, 10' according to the present invention comprises at a first end at least one fixed flange 20, 20' connectable in a proximal position to the respiratory interface 100, 100', and, at an opposite end, at least one movable flange 30, 30' in a distal position with respect to the respiratory interface 100, 100', as well as a tubular 40 of flexible plastic material, constrained at opposite ends to the at least one movable flange 30, 30' and the at least one fixed flange 20, 20' respectively.

The at least one fixed flange 20, 20' and the at least one movable flange 30, 30' both comprise mutual engagement means 50 in a closed position.

The access device 10, 10' is switchable by torsion effect of the at least one movable flange 30, 30' about the central axis thereof from an open position to a closed position, wherein the tubular 40 is arranged in a plane orthogonal to the central axis to form a watertight diaphragm 140.

The mutual engagement means 50 comprise a cylindrical portion 51, 51' of the at least one fixed flange 20, 20' and a cylindrical portion 52, 52' of the at least one movable flange 30, 30', suitable for being mutually superimposed and obtaining a mechanical interlocking.

The mechanical interlocking may, for example, be realised as a threaded connection, bayonet connection or by interference fit, the latter being obtained by means of contact portions having a comparable diameter respectively placed on the cylindrical portions of the at least one fixed flange 20, 20' and of the at least one movable flange 30, 30'.

In the access device 10, 10' according to both embodiments the tubular 40 is fixedly or removably constrained to the fixed flange 51, 51' and to the movable flange 52, 52' along a cylindrical wall 61, 61', 62, 62' constraining the tubular 40.

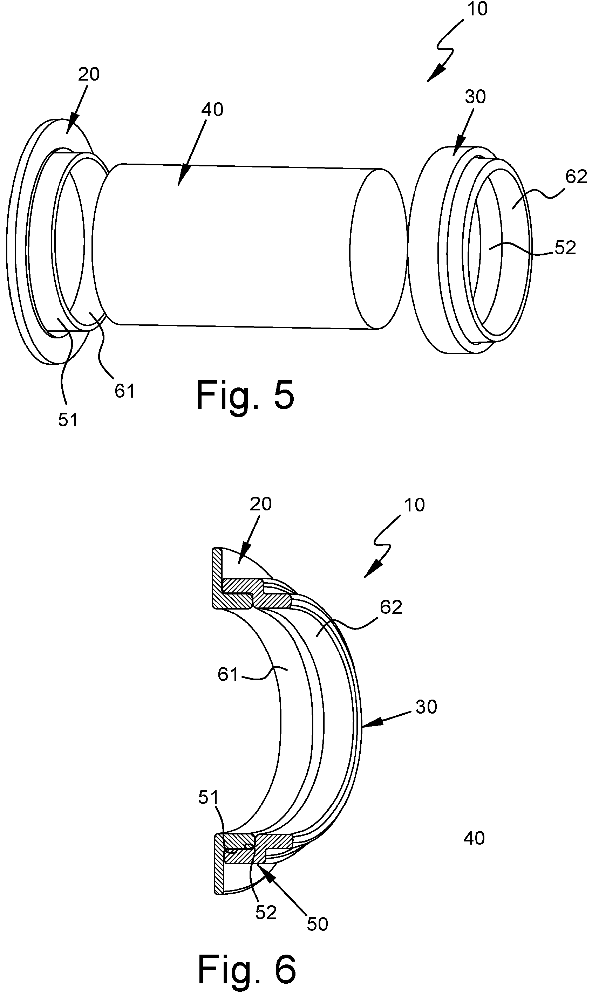

According to the first embodiment, the access device 10, shown in figures 5 and 6, is provided with a single fixed flange 20 and a single movable flange 30.

The tubular 40 is firmly constrained, in particular by gluing or welding, to the cylindrical walls 61 and 62, which are internally arranged on the fixed flange 20 and on the movable flange 30, respectively, having the same diameter.

In the first embodiment of the access device 10, according to the invention the cylindrical portion 52 of the movable flange 30 superimposes on the cylindrical portion 51 of the fixed flange 20 obtaining the mutual mechanical engagement along the contact portion. The outer wall of the cylindrical portion 51 of the fixed flange 20 has a diameter comparable to and interfering with that of the internal wall of the cylindrical portion 52 of the movable flange 30.

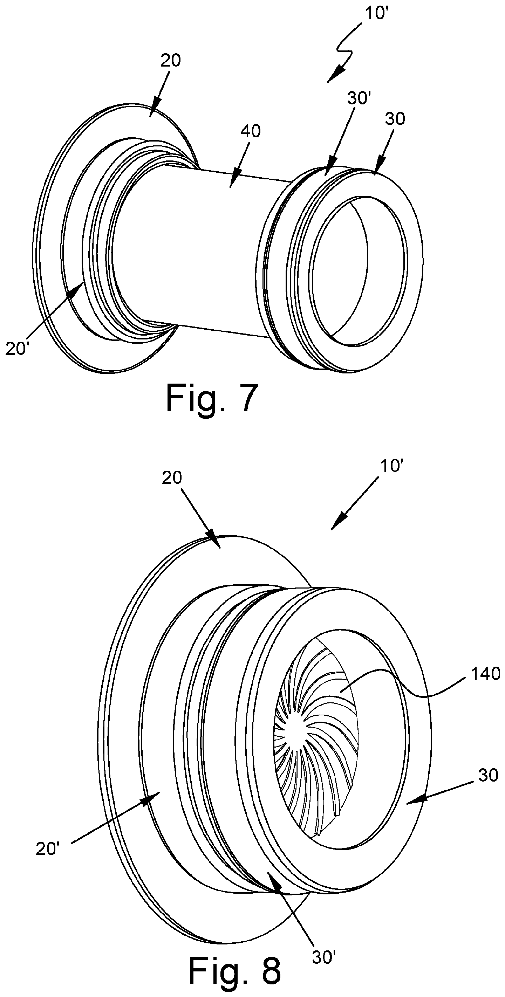

According to the second embodiment of the invention, the access device 10', shown in figures 7 and 8, further comprises a second fixed flange 20' and a second movable flange 30' which are intended to removably constrain the tubular 40 to the first fixed flange 20 and the first movable flange 30.

The second fixed flange 20' and the second movable flange 30' are respectively coupled to the respective cylindrical constraint walls 61', 62' of the tubular 40 present on the first fixed flange 20 and on the first movable flange 30.

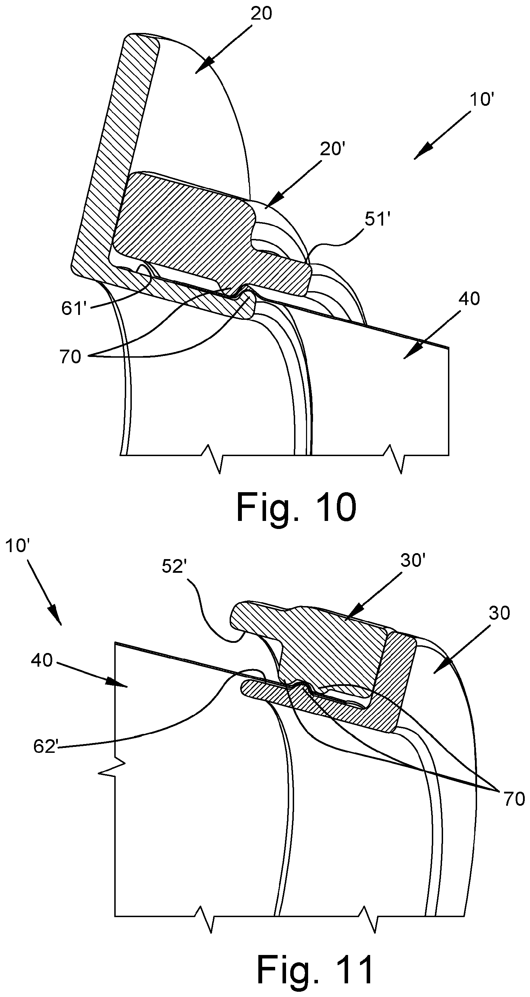

The tubular 40 of flexible plastic material is interposed and interlocked at its ends between the pairs of fixed flanges 20, 20' and the pairs of movable flanges 30, 30' (figures 10 and 11).

For this purpose, the two movable flanges 30, 30' and the two fixed flanges 20, 20' comprise toroidal ribs 70 for mutual snap engagement.

In the second embodiment of the access device 10', according to the invention the cylindrical portion 52' of the movable flange 30' superimposes on the cylindrical portion 51' of the fixed flange 20' obtaining the mutual mechanical engagement along the contact portion. The outer wall of the cylindrical portion 51' of the fixed flange 20' has a diameter comparable to and interfering with that of the internal wall of the cylindrical portion 52' of the movable flange 30'.

The diameter of the tubular 40 is such as to allow an easy passage of the connection fitting for the nasogastric tube, or other accessories. Preferably the internal diameter of the tubular is approximately 35 mm.

The tubular 40, for example, is made of polyurethane, which is particularly suitable as it is very flexible.

By acting on the movable flange 30, 30' with a rotating movement, the operator causes the opening/closing of the access device 10, 10' thus carrying out a diaphragm valve.

The closing and the opening of the access device 10, 10' occurs by interlocking the movable flange 30, 30' and the fixed flange 20, 20' placed on the respiratory interface 100, 100'.

This closing system may also be replaced by other mutual engagement means, such as a thread or other system suitable to cause it to close tightly.

Closing the valve ensures the subsequent pneumatic sealing of the system and therefore the continuation or application of the ventilation therapy without pressure drop, significantly improving the patient's comfort and avoiding "synchronism" and "autotrigger" problems during the therapy.

The application of the access device 10 to the respiratory interface 100, 100' may be permanent or removable.

According to what is shown by way of example, the fixed flange is permanently connected to the respiratory interface 100, for example by welding or gluing.

According to the invention, the fixed flange may be removably connected to the respiratory interface, for example by mechanical interlocking in a fitting of suitable size arranged for that purpose on the respiratory interface at any point.

The access device for a catheter and/or a nasogastric tube for a medical device for non-invasive ventilation which is the object of the present invention has the advantage of allowing the passage of large accessories without preventing the pneumatic sealing of the system.

A further advantage of the access device according to the invention is that it is suitable to be positioned either on a helmet or on a mask, either permanently or as a removable accessory.

Advantageously, the access device according to the invention facilitates the operations of nursing, weaning and/or feeding the patient and setting up the ventilation system, improving the patient's comfort.

The scope of the present invention is limited by the scope of the appended claims.

## Claims

1. Access device for a catheter and/or a nasogastric tube for a respiratory interface comprising, on a first end, at least one fixed flange (20, 20') connectable to a respiratory interface, and, on the opposed end, at least one movable flange (30, 30'), as well a tubular (40) of flexible plastic material, respectively constrained on opposite sides to said at least one movable flange (30, 30') and to said at least one fixed flange (20, 20'), wherein said at least one fixed flange (20, 20') and said at least one movable flange (30, 30') comprise mutual engagement means (50), **characterised in that** the access device is switchable by the rotation of said at least one movable flange (30, 30') about its central axis from an open position to a closed position, in which said tubular (40) is arranged by torsion effect along a plane orthogonal to the central axis to form a watertight diaphragm (140), and **in that** the at least one fixed flange (20, 20') and the at least one movable flange (30, 30') both comprise a cylindrical wall (61, 61', 62, 62') restraining the tubular (40), the access device further comprising a second fixed flange (20') and a second mobile flange (30') respectively coupled to the cylindrical constraint walls (61', 62') of the tubular (40) provided on at least a fixed flange (20) and on the at least one movable flange (30), the tubular (40) of flexible plastic material being interposed and held by interlocking between them.

2. Access device according to claim 1, **characterized in that** the mutual engagement means (50) comprise a cylindrical portion (51, 51') of said at least one fixed flange (20, 20') and a cylindrical portion (52, 52') ) of said at least one movable flange (30, 30 '), suitable for being mutually superimposed and obtaining a mechanical interlocking.

3. Access device according to claim 2, **characterized in that** said cylindrical portions (51, 51', 52, 52' ) have a contact portion having a comparable diameter.

4. Access device according to any one of claims 1 to 3, **characterized in that** it comprises a single fixed flange (20) and a single movable flange (30), the tubular (40) being stably constrained on an internal diameter of the cylindrical walls (61, 62) for restraining the tubular **(40),** for example by welding or gluing.

5. Access device according to claim **4, characterized in that** the two movable flanges (30, 30') and the two fixed flanges (20, 20') include toroidal ribs (70) for mutual snap engagement.

6. Access device according to any one of the preceding claims, **characterized in that** the tubular (40) has an internal diameter equal to about 35mm.

7. Access device according to any one of the preceding claims, **characterized in that** the tubular (40) is made up of polyurethane.

8. Respiratory interface (100, 100') comprising a respiratory chamber (101, 101') that can be positioned on the head or face of a patient, an air inlet and an air outlet, **characterized in that** it comprises an access device of a catheter and/or nasogastric tube (10, 10') according to any one of the preceding claims.

## Patentansprüche

1. Zugangsvorrichtung für einen Katheter und/oder eine nasogastrale Sonde für eine Atemschnittstelle, die an einem ersten Ende mindestens einen festen Flansch (20, 20') aufweist, der mit einer Atemschnittstelle verbunden werden kann, und am gegenüberliegenden Ende mindestens einen beweglichen Flansch (30, 30') sowie ein Rohrstück (40) aus flexiblem Kunststoff, das jeweils an gegenüberliegenden Seiten an dem mindestens einen beweglichen Flansch (30, 30') und an dem mindestens einen feststehenden Flansch (20, 20') befestigt ist, wobei der mindestens eine feststehende Flansch (20, 20') und der mindestens eine bewegliche Flansch (30, 30') gegenseitige Eingriffsmittel (50) aufweisen, **dadurch gekennzeichnet, dass** die Zugangsvorrichtung durch Drehen des mindestens einen beweglichen Flansches (30, 30') um seine Mittelachse aus einer offenen Position in eine geschlossene Position umschaltbar ist, in der das Rohrstück (40) durch Torsionswirkung entlang einer zur Mittelachse orthogonalen Ebene angeordnet ist, um eine wasserdichte Membran (140) zu bilden, und dass der mindestens eine feste Flansch (20, 20') und der mindestens eine bewegliche Flansch (30, 30') beide eine zylindrische Wand (61, 61', 62, 62') aufweist, die das Rohr (40) zurückhalten, wobei die Zugangsvorrichtung ferner einen zweiten feststehenden Flansch (20') und einen zweiten beweglichen Flansch (30') umfasst, die jeweils mit den zylindrischen Rückhaltewänden (61', 62') des Rohrs (40) verbunden sind, das an mindestens einem festen Flansch (20) und an dem mindestens einen beweglichen Flansch (30) vorgesehen ist, wobei das Rohr (40) aus flexiblem Kunststoffmaterial dazwischen angeordnet und durch Verriegelung zwischen ihnen gehalten wird.

2. Zugangsvorrichtung nach Anspruch 1, **dadurch gekennzeichnet, dass** die gegenseitigen Eingriffsmittel (50) einen zylindrischen Abschnitt (51, 51') des mindestens mindestens einen festen Flansch (20, 20') und einen zylindrischen Abschnitt (52, 52') des mindestens einen beweglichen Flansches (30, 30'), die geeignet sind, sich gegenseitig zu überlagern und eine mechanische Verriegelung zu erzielen.

3. Zugangsvorrichtung nach Anspruch 2, **dadurch gekennzeichnet, dass** die zylindrischen Abschnitte (51, 51', 52, 52') einen Kontaktabschnitt mit einem vergleichbaren Durchmesser aufweisen.

4. Zugangsvorrichtung nach einem der Ansprüche 1 bis 3, **dadurch gekennzeichnet, dass** sie einen einzigen festen Flansch (20) und einen einzigen beweglichen Flansch (30) umfasst, wobei das Rohr (40) stabil auf einem Innendurchmesser der zylindrischen Wände (61, 62) eingespannt ist, um das Rohr (40) zum Beispiel durch Schweißen oder Kleben festzuhalten.

5. Zugangsvorrichtung nach Anspruch 4, **dadurch gekennzeichnet, dass** die beiden beweglichen Flansche (30, 30') und die beiden festen Flansche (20, 20') toroidale Rippen (70) zum gegenseitigen Einrasten aufweisen.

6. Zugangsvorrichtung nach einem der vorhergehenden Ansprüche, **dadurch gekennzeichnet, dass** das Rohr (40) einen Innendurchmesser von etwa 35mm aufweist.

7. Zugangsvorrichtung nach einem der vorhergehenden Ansprüche, **dadurch gekennzeichnet, dass** der Schlauch (40) aus Polyurethan besteht.

8. Atmungsschnittstelle (100, 100') mit einer Atmungskammer (101, 101'), die auf dem Kopf oder dem Gesicht eines Patienten positioniert werden kann, einem Lufteinlass und einem Luftauslass, **dadurch gekennzeichnet, dass** sie eine Zugangsvorrichtung umfasst eines Katheters und/oder einer nasogastrischen Sonde (10, 10') nach einem der vorhergehenden Ansprüche.

## Revendications

1. Dispositif d'accès pour un cathéter et/ou une sonde nasogastrique pour une interface respiratoire comprenant, sur une première extrémité, au moins une bride fixe (20, 20') pouvant être raccordée à une interface respiratoire, et, sur l'extrémité opposée, au moins une bride mobile (30, 30'), ainsi qu'un tube (40) en matière plastique souple, respectivement contraint sur les côtés opposés à ladite au moins une bride mobile (30, 30') et à ladite au moins une bride fixe (20, 20'), dans lequel ladite au moins une bride fixe (20, 20') et ladite au moins une bride mobile (30, 30') comprennent des moyens d'engagement mutuel (50), **caractérisé en ce que** le dispositif d'accès est commutable par la rotation de ladite au moins une bride mobile (30, 30') autour de son axe central d'une position ouverte à une position fermée, dans laquelle ledit tube (40) est disposé par effet de torsion le long d'un plan orthogonal à l'axe central pour former un diaphragme étanche (140), et **en ce que** la au moins une bride fixe (20, 20') et la au moins une bride mobile (30, 30') comprennent toutes deux une paroi cylindrique (61, 61', 62, 62') retenant le tube (40), le dispositif d'accès comprenant en outre une deuxième bride fixe (20') et une deuxième bride mobile (30') respectivement couplées aux parois de contrainte cylindriques (61', 62') du tube (40) prévues sur au moins une bride fixe (20) et sur la au moins une bride mobile (30), le tube (40) en matière plastique flexible étant interposé et maintenu par emboîtement entre elles.

2. Dispositif d'accès selon la revendication 1, **caractérisé en ce que** les moyens d'engagement mutuel (50) comprennent une partie cylindrique (51, 51') de ladite au moins au moins une bride fixe (20, 20') et une partie cylindrique (52, 52') de ladite au moins une bride mobile (30, 30'), aptes à se superposer mutuellement et à obtenir un emboîtement mécanique.

3. Dispositif d'accès selon la revendication 2, **caractérisé en ce que** lesdites parties cylindriques (51, 51', 52, 52') ont une partie de contact ayant un diamètre comparable.

4. Dispositif d'accès selon l'une quelconque des revendications 1 à 3, **caractérisé en ce qu'**il comprend une seule bride fixe (20) et une seule bride mobile (30), le tube (40) étant contraint de manière stable sur un diamètre intérieur des parois cylindriques (61, 62) pour retenir le tube (40), par exemple par soudage ou collage.

5. Dispositif d'accès selon la revendication 4, **caractérisé en ce que** les deux brides mobiles (30, 30') et les deux brides fixes (20, 20') comprennent des nervures toroïdales (70) pour un engagement mutuel par encliquetage.

6. Dispositif d'accès selon l'une quelconque des revendications précédentes, **caractérisé en ce que** la tubulaire (40) a un diamètre interne égal à environ 35mm.

7. Dispositif d'accès selon l'une quelconque des revendications précédentes, **caractérisé en ce que** la tubulaire (40) est constituée de polyuréthane.

8. Interface respiratoire (100, 100') comprenant une chambre respiratoire (101, 101') pouvant être positionnée sur la tête ou le visage d'un patient, une entrée d'air et une sortie d'air, **caractérisée en ce qu'**elle comprend un dispositif d'accès d'un cathéter et/ou d'une sonde nasogastrique (10, 10') selon l'une quelconque des revendications précédentes.
